# EUROPÄISCHE PATENTSCHRIFT

(11) **EP 0 927 561 B1**
(45) Veröffentlichungstag und Bekanntmachung des Hinweises auf die Patenterteilung: **11.05.2005**
(21) Anmeldenummer: 98250429.2
(22) Anmeldetag: 04.12.1998
(51) Int. Cl.: A61N 1/39, A61N 1/05

(54) **Defibrillationselektrodenanordnung**
Defibrillation electrode device
Système d'électrodes de défibrillation

(30) Priorität: 29.12.1997 DE 19758368
(43) Veröffentlichungstag der Anmeldung: 07.07.1999
(73) Patentinhaber: BIOTRONIK Mess- und Therapiegeräte GmbH & Co Ingenieurbüro Berlin, 12359 Berlin (DE)
(72) Erfinder: Belke, Roberto, Dipl.-Ing., 22119 Hamburg (DE); Niehaus, Michael, Dr. med., 30659 Hannover (DE); Bartels, Klaus, Dr. rer. nat., 10115 Berlin (DE)
(74) Vertreter: Eisenführ, Speiser & Partner

(56) Entgegenhaltungen:
- EP-A- 0 442 444
- US-A- 3 788 329
- US-A- 4 964 414
- US-A- 5 203 348
- US-A- 5 439 485
- US-A- 5 483 022

## Beschreibung

Die Erfindung betrifft eine Defibrillationselektrodenanordnung gemäß dem Oberbegriff des Anspruchs 1 sowie ein Verfahren zur Herstellung einer derartigen Elektrode.

Implantierbare automatische Defibrillatoren finden zunehmend Einsatz bei Patienten mit medikamentös nicht oder nicht zuverlässig therapierbaren Herzrhythmusstörungen, die zu einem plötzlichen Auftreten von Herzkammerflimmern führen können. Solche Patienten bedürfen in vielen Fällen zusätzlich einer Schrittmacherunterstützung ihrer Herztätigkeit, so daß speziell auch Kombinationsgeräte mit einem Herzschrittmacher einige Verbreitung erlangt haben.

Wegen des geringeren Operationsaufwandes und -risikos setzen sich als Elektrodenanordnungen gegenüber den früheren subkutanen Flächenelektroden immer deutlicher solche Anordnungen durch, bei denen zumindest ein Teil der Elektroden auf einem transvenös endokardial zu verlegenden Katheter- bzw. Elektrodenleitungskörper angeordnet ist. Diese Elektrodenanordnungen haben in Verbindung mit Schrittmacher/Defibrillator-Kombinationsgeräten zudem den Vorteil, daß ein Teil der Elektroden sowohl als Schrittmacher- wie auch als Defibrillationselektroden genutzt werden kann oder jedenfalls Abfühl-, Schrittmacher- und Defibrillationselektroden auf ein und derselben Elektrodenleitung ausgeführt werden können, so daß das Verlegen mehrerer separater Katheter weitgehend unnötig wird.

Die bekannten Defibrillationselektrodenanordnungen sind entweder - sofern sie einen einfachen Aufbau haben und leicht implantierbar sind - hinsichtlich ihrer Funktionalität relativ eingeschränkt, oder sie sind soweit sie komplexere Funktionen erfüllen sollen - kompliziert aufgebaut und sowohl aufwendig in der Herstellung als auch in der Implantation.

Aus der US-Patentschrift 5 203 348 ist eine Defibrillationselektrode für die subkutane Applikation in Form einer großflächig ausgebildeten Spirale bekannt, welche das distale Ende der Zuleitung der Defibrillationselektrode bildet.

Die Zuleitung der Defibrillationselektrode weist eine elektrisch isolierende Schlauchhülle auf und ist als Einzelstrang oder als seilartige Leitung aus mehreren gleichartigen Einzelsträngen ausgebildet, welche nach dem Drawn-Filled-Tubing-Verfahren (DFT-Verfahren) hergestellt sind.

Die einzelnen Adern des die Spirale bildenden Leitungsstrangs (nachstehend als DFT-Seil bezeichnet) weisen einen Kern aus Silber auf, welcher eine äußere Lage aus einem mit Nickel, Kobalt, Chrom und Molybdän legiertem Stahl (Handelsname MP35N-Stahl) trägt.

Aus der US 3,788,329 ist eine Elektrodenleitung bekannt, bei der ein Leiter aus einer isolierenden Hülle herausgeführt und um die isolierende Hülle herumgewickelt ist, so dass sich eine Wendelelektrode bildet.

Aus der US 5,483,022 ist ein Leiter für eine Elektrodenleitung bekannt, dessen Filamente als DFT-Seile ausgebildet sind.

Der Erfindung liegt deshalb die Aufgabe zugrunde, eine Defibrillationselektrodenanordnung der eingangs genannten Gattung anzugeben, welche für intrakardiale Anwendungen zur Implantation geeignet ist und dort eine große Wechsellastbeständigkeit aufweist.

Die Aufgabe wird durch die im kennzeichnenden Teil des Anspruchs 1 angegebenen Merkmale gelöst.

Die Erfindung schließt die Erkenntnis ein, daß besondere Vorteile bei der Fertigung einer wendelförmigen Elektrodenanordnung, insbesondere einer Defibrillationselektrode, erreichbar sind, wenn die Wendel auf einem Träger in Fortsetzung der Zuleitung ohne mechanische Verbindungs- und Kontaktierungsmittel für das DFT-Seil erzeugt werden kann. Auf diese Weise besteht eine große Ausfallsicherheit für die Elektrode, da aufgrund der Homogenität der Zuleitung auch bei großer mechanischer Belastung ein Bruch nicht zu befürchten ist. Es läßt sich eine hohe Wechsellastbeständigkeit ohne Vergrößerung des Elektrodendurchmessers erzielen.

Des weiteren sind keine zusätzlichen Mittel für die Aufrechterhaltung der mechanischen Stabilität der Wendel erforderlich, wenn die Ummantelung der elektrischen Elektrodenzuleitung oder ein dieses in axialer Richtung fortsetzendes Element zum Stabilisieren einer gewendelten Defibrillationselektrode genutzt wird. In diesem Fall wird auf ein und demselben Körper die galvanische Elektrodenzuleitung zunächst isolierend und vor Stromverlusten geschützt im Innern der Ummantelung geführt, während sie im aktiven Bereich ohne Unterbrechung auf die Außenseite des Trägers gelangt, um hier in implantiertem Zustand intrakardial mit dem Herzen in elektrische Wechselwirkung zu treten.

Darüber hinaus sind in günstiger Weise Kontaktierungsprobleme bei der Verwendung eines DFT-Seils als elektrische Zuleitung für eine Defibrillationselektrode insbesondere dann vermieden, wenn diese unmittelbar aus dem distalen Ende der Zuleitung durch einen Durchbruch herausgeführt wird um sich dann als zylindrischer Bereich frei zur galvanischen Kontaktierung der Umgebung fortzusetzen. Hierbei ist als Träger für den aktiven Bereich der Elektrode auch jeder zylindrische Körper geeignet, der sich an die Ummantelung der Zuleitung in geeigneter Weise anschließt und insbesondere auch eine geeignete Oberflächengestaltung (Ausnehmungen zur Aufnahme der Wendelung) aufweisen kann. Wichtig ist hier also die direkte galvanische Fortsetzung der elektrischen Zuleitung, welche auch den elektrisch aktiven Stimulationsbereich bildet, wobei ein Verfahren zur Herstellung darin besteht, dass die DFT-Wendel distal freigelegt und um einen zylindrischen Körper herumgewickelt wird, welcher die Ummantelung der Zuleitung - insbesondere ebenfalls einstückig - fortsetzt.

Entsprechend einer bevorzugten Ausführungsform der Erfindung weist die Defibrillationselektrodenanordnung eine intrakardial positionierbare Elektrodenleitung mit einer Defibrillationselektrode und weiteren, zum Abtasten und/oder Stimulieren von ventrikulärem Gewebe vorgesehenen Elektroden auf, deren Zuleitungen in einer gemeinsamen Schlauchhülle geführt sind. In der Schlauchhülle ist für jede der Zuleitungen ein separates Lumen vorgesehen.

Die Defibrillationselektrode ist als Schraubenwendel ausgebildet, wobei das distale Ende der Zuleitung der Defibrillationselektrode aus dem für die Zuleitung vorgesehenen Lumen durch die Wandung der Schlauchhülle nach außen geführt und um die Schlauchhülle gewunden ist.

Die Schlauchhülle bildet dabei einen die Schraubenwendel tragenden Kern, wobei zwischen den einzelnen Windungen der Schraubenwendel und der Oberfläche der Schlauchhülle eine reibschlüssige Verbindung besteht. Die den Kern bildende Schlauchhülle gibt der Schraubenwendel somit eine ausreichende axiale Führung bzw. Form- und Lagestabilität, wenn eine mechanische Belastung der Schraubenwendel, beispielsweise bei einer Implantation, eintritt.

Entsprechend einer günstigen Weiterbildung der Erfindung kann die Form- und Lagestabilität der Schraubenwendel verbessert werden, indem in die Mantelfläche der Schlauchhülle im Bereich der Defibrillationselektrode eine wendelförmige Nutung eingearbeitet ist. Die Nuten werden dabei durch die einzelnen Windungen der Schraubenwendel zumindest teilweise ausgefüllt. Die daraus resultierende Vergrößerung der Kontaktfläche zwischen Schlauchhülle und den einzelnen Windungen sichert auch bei Einwirken stärkerer axialer Kraftkomponenten auf die Defibrillationselektrode einen festen, lagestabilen Sitz der Wendel.

In einer vorteilhaften Variante der Erfindung ist eine Defibrillationselektrode vorgesehen, bei welcher das zur Bildung der Defibrillationselektrode verwendete distale Ende des DFT-Seils vom Kopf der Elektrode her gewendelt ist. Das DFT-Seil ist dazu innerhalb des Innenraums der Schraubenwendel geführt, wobei die Innenseite der einzelnen Windungen der Schraubenwendel das DFT-Seil der Zuleitung unter Bildung jeweils eines lokalen elektrischen Kontakts berührt.

Durch eine derartige Kontaktgabe ist auf einfache Weise eine Vergleichmäßigung der Stromdichteverteilung an der Oberfläche der Defibrillationselektrode erreichbar.

Nach einer weiteren vorteilhaften Variante der Erfindung weist das DFT-Seil im Windungsbereich der Defibrillationselektrode einen von der Kreisform abweichendes Querschnittsprofil auf. Ein solches Querschittsprofil sichert eine größere Kontaktfläche zwischen Defibrillationselektrode zu stimulierendem Körpergewebe, wodurch die gleiche medizinische Wirkung mit einer geringeren elektrischen Leistung erreicht werden kann.

Entsprechend einer günstigen Weiterbildung der Erfindung sind die Lumina für die Zuleitungen der einzelnen Elektroden innerhalb der Schlauchhülle aus Kunststoff - bezogen auf Längsachse der Schlauchhülle - außermittig derart angeordnet, daß sowohl der kleinste Abstand zwischen der Mantelfläche der Schlauchhülle und der Mantelfläche der einzelnen Lumina als auch der kleinste Abstand der Mantelflächen jeweils benachbart angeordneter Lumina im wesentlichen den selben Wert aufweisen.

Diese Form der Symmetrie sichert eine im wesentlichen gleichmäßige Verteilung der mechanischen Belastung der Zuleitungen, wenn die Elektrodenleitung bei der Implantation einer Biegebeanspruchung unterworfen wird.

Die Defibrillationselektrodenanordnung ist durch den Einsatz von DFT-Seilen als Elektrodenzuleitung und durch die erfindungsgemäße Ausbildung der Defibrillationselektrode als wendelförmiger Endbereich einer Zuleitung in vorteilhafter Weise mit relativ geringem technologischen Aufwand herstellbar. Bei gleicher mechanischer Festigkeit und gleichzeitig verbesserter Leitfähigkeit können als DFT-Seil ausgebildete Zuleitungen mit verringertem Außendurchmesser eingesetzt werden. Durch die Verwendung von DFT-Seilen sind intrakardial einfacher zu implantierende Elektrodenleitungen mit kleinerem Durchmesser herstellbar und der Umfang der funktionelle Beeinflussung der zwischen Atrium und dem jeweiligen Ventrikel befindlichen Herzklappe durch eine implantierte Defibrillationselektrodenanordnung wird deutlich reduziert.

Eine Durchmesserverringerung ist insbesondere dann vorteilhaft, wenn die Elektrodenanordnung drei- und mehrpolig ausgebildet ist und multifunktional eingesetzt werden soll, um den Implantationsaufwand so gering wie möglich zu halten.

Ein Verfahren zur Herstellung einer Defibrillationselektrodenanordnung zeichnet sich dadurch aus, daß die Ummantelung am distalen Ende der Elektrode um eine Länge gekürzt wird, welche der Länge des Leiters im Bereich der Wendel bzw. der Länge des DFT-Seils als Leiter im Bereich der Wendel vermindert um die axiale Länge der Wendel entspricht, wobei das entsprechende DFT-Seil als Leiter der Zuleitung für seine Länge im Bereich der Wendel freigelegt und dann um einen die Ummantelung in axialer Richtung verlängernden zylindrischen Körper oder um den Restlängenbereich der Ummantelung, aus dem das DFT-Seil als Leiter entfernt wurde, wendelförmig herumgewickelt wird. Dabei werden insbesondere zur Herstellung einer mehrpoligen Elektrode weitere DFT-Seile als elektrische Leiter durch den Mantel der Zuleitung hindurch bzw. einer entsprechenden mechanischen Verlängerung vor dem mit der Wendel zu versehenden Ende durch die Wandung hindurchgeführt und mit aktiven Elektrodenbereichen zur Kontaktierung des Herzens verbunden.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine bevorzugte Ausführungsform der Erfindung in Draufsicht,
- Figur 2: die Darstellung der Ansicht des Schnittes längs der Linie A...A gemäß Figur 1,
- Figur 3: die Darstellung der Ansicht eines Teilschnittes längs der Linie B...B gemäß Figur 1,
- Figur 4: eine vorteilhafte Weiterbildung der in Figur 3 gezeigten Ausführungsform der Erfindung,
- Figur 5: eine zusätzliche Variante der Erfindung in Teilschnittdarstellung,
- Figur 6: eine andere vorteilhafte Ausführungsform der Erfindung in Draufsicht sowie
- Figur 7: die Darstellung der Ansicht des Schnittes längs der Linie C...C gemäß Figur 6.

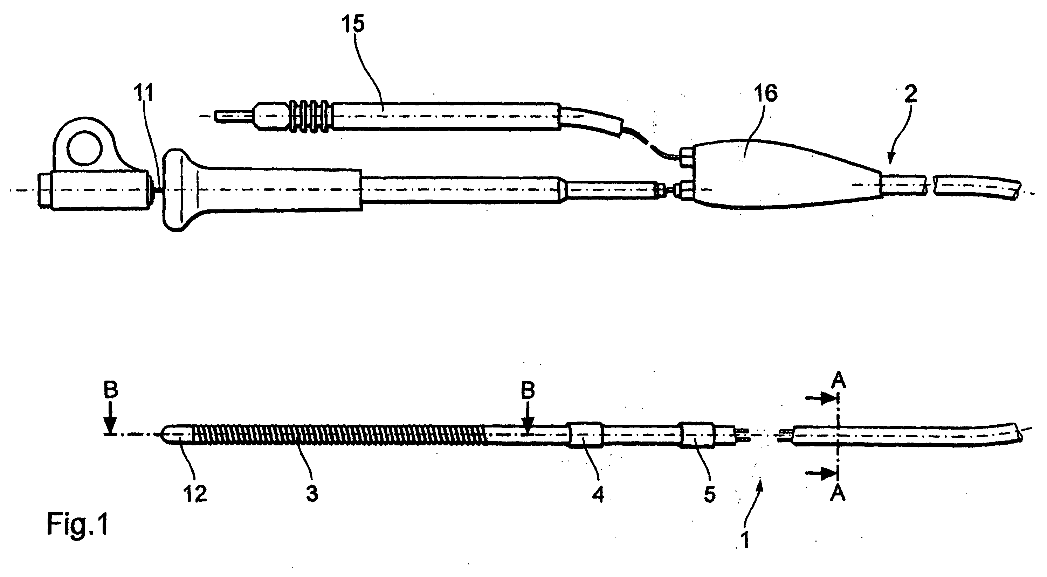

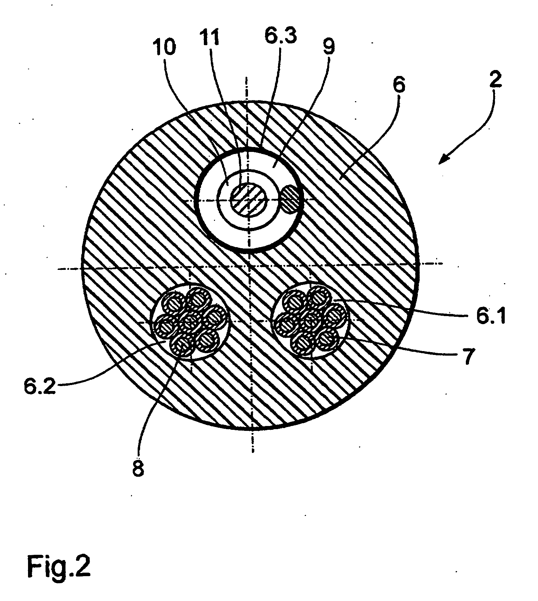

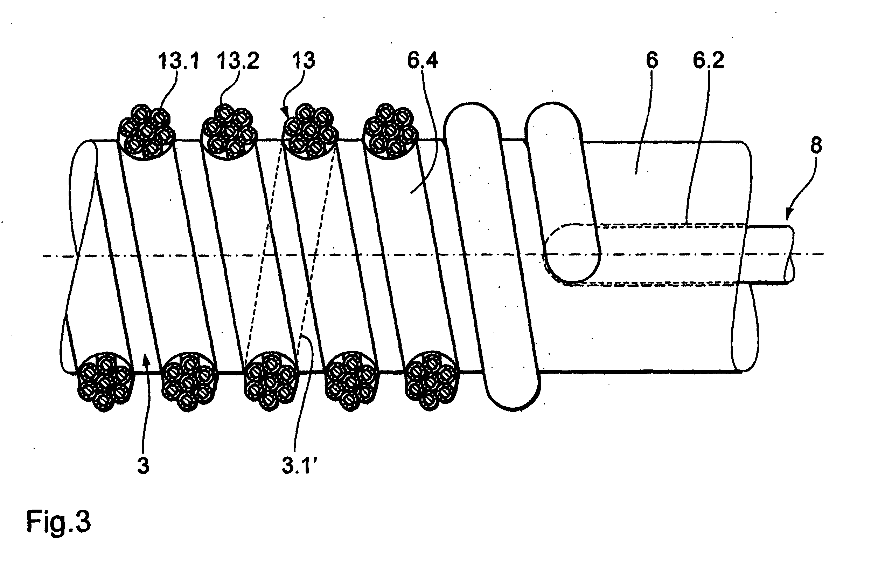

In Figur 1 ist eine Defibrillationselektrodenanordnung (1) dargestellt, von welcher in den Schnittansichten gemäß den Figuren 2 und 3 konstruktive Details gezeigt sind. Aus Gründen der Übersichtlichkeit ist in Figur 3 nur eine Zuleitung dargestellt.

Die in Figur 1 gezeigte Defibrillationselektrodenanordnung (1) weis eine Elektrodenleitung (2) auf, an deren distalen Ende die Elektroden (3, 4, 5) angeordnet sind.

Die Defibrillationselektrode (3) (nachfolgend als Schockwendel bezeichnet) ist als Schraubenwendel ausgebildet. Die Elektroden (4) und (5) weisen dagegen eine Ringform auf und sind für ventrikuläre Abtast- und/oder Stimulationszwecke vorgesehen.

Die Elektrodenleitung (2) weist - wie in Figur 2 gezeigt - eine Schlauchhülle (6) aus Silikonmaterial auf, in welcher die Zuleitungen (7, 8 und 9) jeweils in einem separaten Lumen (6.1, 6.2 und 6.3) angeordnet sind. Die Lumina (6.1, 6.2 und 6.3) bilden zylindrische Kanäle, welche sich parallel zur Achse der Elektrodenleitung (2) erstrecken. Die Lumina (6.1) und (6.2) weisen einen Durchmesser von 0,5 mm auf, für das Lumen (6.3) ist ein Durchmesser von 0,7 mm vorgesehen.

Die Zuleitungen (7) und (8) sind als DFT-Seil ausgebildet und in gestreckter Form in den Lumina (6.1) bzw. (6.2) geführt, während die Zuleitung (9) als herkömmliche MP35N-Stahl-Wendel ausgebildet und in dem Lumen (6.3) plaziert ist.

Die einzelnen Adern (13) der DFT-Seile (7, 8) weisen - wie in Figur 3 gezeigt - einen Kern (13.1) aus Silber auf, welcher eine Ummantelung (13.2) aus Stahl oder aus einer Platin-Iridium-Legierung trägt. Für das DFT-Seil ist eine (nicht dargestellte) Isolation vorgesehen. Der Durchmesser der DTF-Seile (7) und (8) beträgt 0, 27 mm.

Zur Herausbildung der Schraubenwendel (3) ist das distale Ende des DFT-Seils (8) aus dem Lumen (6.2) durch die Schlauchhülle (6) herausgeführt und in die an der Peripherie der Schlauchhülle (6) eingearbeitete wendelförmige Nutung (6.4) eingelegt. Die einzelnen Windungen (3.1) der Schockelektrode erstrecken sich parallel zueinander und sind durch einen reibschlüssigen Verbund mit der Schlauchhülle (6) lagesicher angeordnet.

Wie in der Teilschnittdarstellung gemäß Figur 3 gezeigt, bildet das distale Ende der als DFT-Seil ausgebildeten Zuleitung (8) eine angeformte Schockwendel (3), d.h. die Zuleitung (8) geht ohne eine gesonderte Kontaktierung oder mechanische Verbindung zur Überbrückung einer Trennstelle unmittelbar in den gewendelten Bereich über.

Die MP35N-Stahl-Wendel (9) weist einen Innendurchmesser von 0,4 mm und einen Außendurchmesser von 0,6 mm auf und ist mit der Ringelektrode (4) durch eine Laserschweißung verbunden.

Der im Innern des Lumens (6.3) nach Platzieren der MP35N-Stahl-Wendel (9) verbleibende Freiraum (10) nimmt den zum Implantieren der in ein Ventrikel des Herzens benutzten Führungsdraht (11) auf, welcher bis zu der gegenüber der Schockwendel (3) elektrisch isolierten Metallspitze (12) am distalen Ende der Elektrodenleitung (2) reicht.

Die als DFT-Seil ausgebildete Zuleitung (7) im Lumen (6.1) ist der Ringelektrode (5) zugeordnet und mit dieser durch eine Widerstandsschweißung verbunden. Die Ringelektroden (4) und (5) weisen jeweils einen Außendurchmesser von 0,42 mm auf.

Durch Fortfall der nur mit einem erhöhten technologischen Aufwand herstellbaren Kontaktstelle zwischen der Schockelektrode (3) und der aus einemm DFT-Seil gebildeten Zuleitung wird gesichert, daß der Defibrillationsimpuls frei von Verlusten durch leitungsbedingte Übergangswiderstände auf das zu stimulierende Körpergewebe übertragen werden kann und somit in günstiger Weise nur eine geringere elektrische Leistung durch den vorgesehenen Defibrillator bereitzustellen ist.

Im distalen Bereich der Zuleitung ist die Isolation von dem DFT-Seil (8) entfernt, um eine gute Kontaktbildung zwischen der fünfzehn Windungen (3.1) aufweisenden Schockwendel (3) und dem zu stimulierenden Gewebe zu sichern. Die Schockwendel (3) weist im Vergleich zu einer herkömmlichen, aus einem Stahlband gewickelten Defibrillationselektrode durch die Verwendung eines DFT-Seils zur Herstellung der einzelnen Windungen (3.1) der Schraubenwendel eine vergrößerte Oberfläche zur Kontaktierung des zu stimulierenden Körpergewebes auf.

Zur Vereinfachung der Darstellung ist die Zuleitung (8) in Figur 3 nur teilweise in Seilstruktur gezeigt.

Die Defibrillationselektrodenanordnung (1) ist durch den Einsatz der DFT-Seile (7, 8) und durch die Ausbildung der Schokkelektrode (3) als wendelförmiger Endbereich einer Zuleitung (8) ein vorteilhafter Weise - im Vergleich zu einer herkömmlichen Löt- oder Schweißverbindung - wegen der fehlenden Kontaktstellen mit geringerem technologischen Aufwand herstellbar. Gleichzeitig ist die Bruchgefahr an einer Kontaktstelle zwischen Zuleitung und der dazugehörigen Elektrode ausgeschlossen. Darüberhinaus weisen die DFT-Seile ausgebildeten Zuleitungen (7) und (8) bei gleicher mechanischer Festigkeit sowie gleichzeitig verbesserter Leitfähigkeit einen verringerten Außendurchmesser auf, so daß die Elektrodenleitung (2) mit einem Außendurchmesser von nur 2,2 mm herstellbar ist.

Als Gegenelektrode für die Schockwendel (3) wird - abhängig von den patientenspezifischen Bedingungen - entweder eine gesondert zu implantierende Elektrode oder das metallische Gehäuse des Defibrillators verwendet.

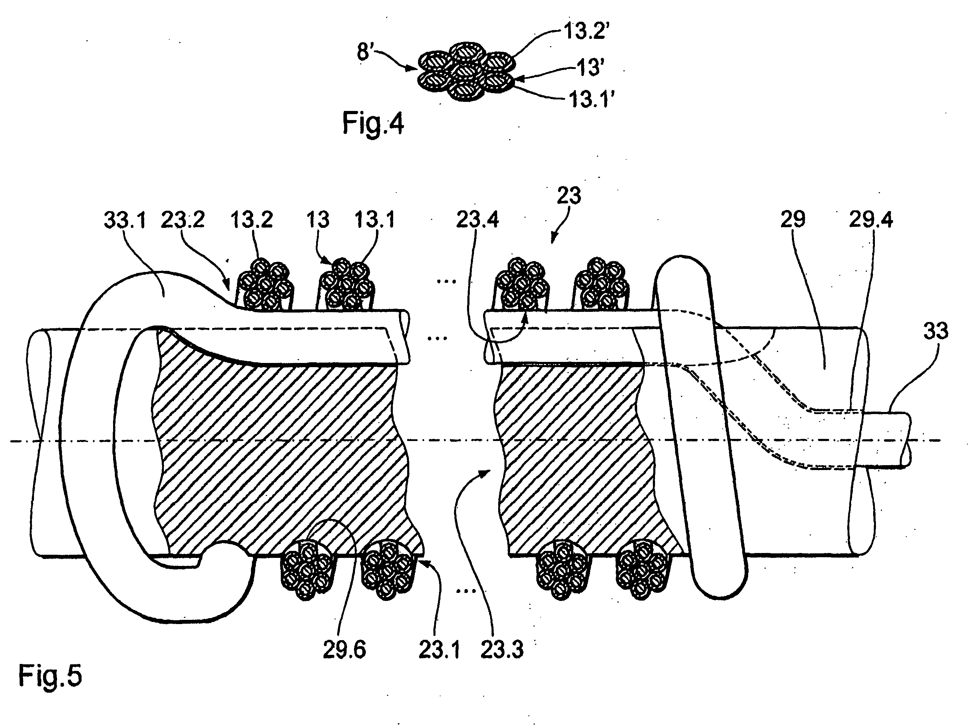

In Figur 4 ist die Querschnittsansicht des distalen Endes eines DFT-Seils (8') mit einem von der Kreisform abweichenden Querschnittsprofil. Die einzelnen Adern (13') weisen einen eliptischen Kern (13.1') mit einer eliptischen Mantelung (13.2'), so daß bei einer aus diesem Seilabschnitt geformten Schraubenwendel bei gleichem Wendeldurchmesser eine größere Kontaktfläche zur Stimulation des Körpergewebes zur Verfügung steht, als bei einer aus einem Seil mit kreisförmigen Querschnitt geformten Defibrillationselektrode.

Die in Figur 5 in Teilschnittdarstellung gezeigte Defibrillationselektrode (23) (nachfolgend als Schockwendel bezeichnet) ist als Schraubenwendel ausgebildet und weist eine Wendelung auf, welche am Elektrodenkopf (23.2) beginnt. Dazu wird das distale Ende der Zuleitung (33) aus dem Lumen (29.4) heraus- und achsparallel weitergeführt und dann um die Schlauchhülle (29) herum in Richtung des proximalen Endes der Elektrodenleitung gewickelt. Dabei wird ein Zuleitungsabschnitt (33.1) von den Windungen (23.1) der Schockwendel (23) umfaßt und verbleibt nach Bildung der Wendel im inneren Hohlraum (23.3) der Schockwendel (23). Die in der Schlauchhülle (29) im Bereich der Schockwendel (23) vorgesehene Nutung ist mit (29.6) bezeichnet. Die einzelnen Windungen (23.1) der Schockwendel (23) liegen in den im Mantel der Schlauchhülle (29) eingearbeiteten Nuten und sichern einen festen Sitz der Schockwendel auf der den Wickelkern bildenden Schlauchhülle. Der Aufbau der Zuleitung (33) entspricht dem der in Figur 3 beschriebenen DFT-Seile (7) und (8).

Aus Gründen der Übersichtlichkeit wurde in Figur 5 nur die Zuleitung (33) gezeigt und auf die Darstellung aller vorhandenen Leitungen verzichtet.

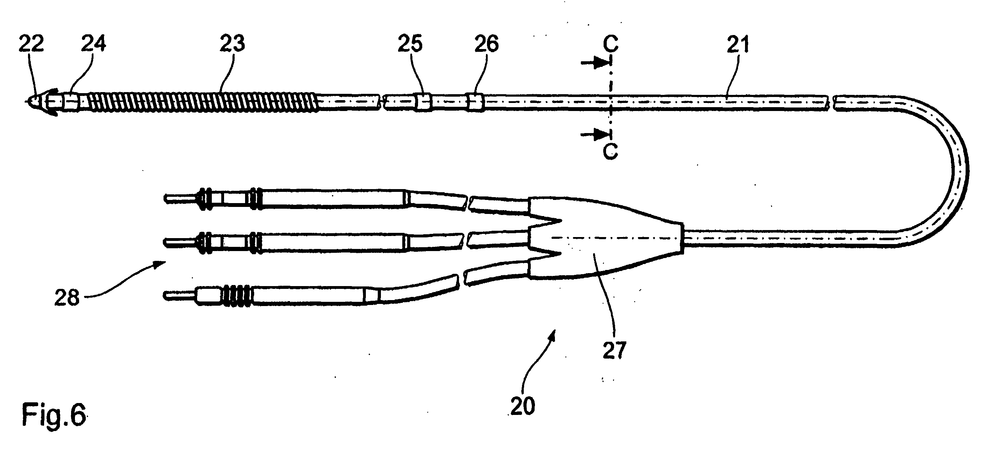

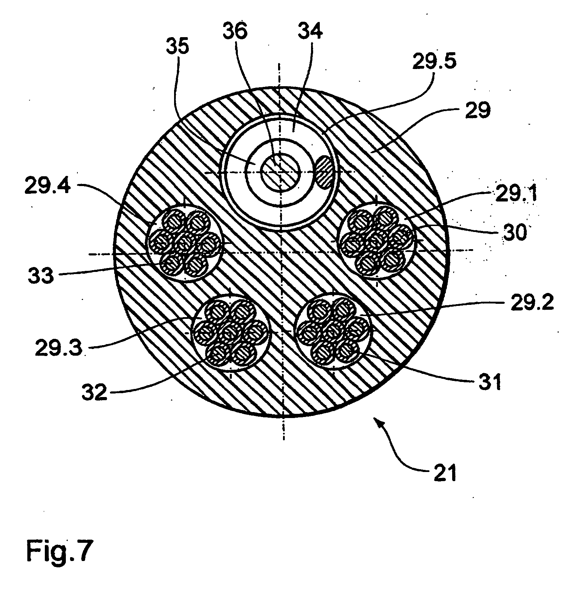

Figur 6 zeigt eine Defibrillatorelektrodenanordnung (20) mit einer fünfpoligen Elektrodenleitung (21) mit einem Außendurchmesser von 2,6 mm, deren Querschnittsansicht in Figur 7 dargestellt ist.

An der Elektrodenleitung (21) sind die Elektroden (22) und (24) sowie die Schockwendel (23) für eine ventrikuläre Abfühl- bzw. Stimulationsfunktion und die Ringelektroden (25) und (26) für eine atriale Abfühl- bzw. Stimulationsfunktion vorgesehen. Die Zuleitungen (30 bis 34) der Elektroden (22 bis 26) sind in sich parallel zueinander erstreckenden, zylindrischen Lumen (29.1 bis 29.5) der aus Silikonmaterial bestehenden Schlauchhülle (29) angeordnet.

Mit Ausnahme der am distalen Ende der Elektrodenleitung (21) befindlichen Elektrode (22), welche eine Stahlwendel (34) als Zuleitung aufweist, sind die Zuleitungen (30 bis 33) als siebenadriges DFT-Seil ausgebildet, so daß der Außendurchmesser der Elektrodenleitung (21) in günstiger Weise einen Wert von nur 2,6 mm aufweist.

Der Aufbau der Schockwendel (23) ist in Figur 5 detailiert dargestellt. Über den Verteiler (27) sind die in der Elektrodenleitung vorhandenen Zuleitungen (30 bis 35) einzeln bzw. paarweise mit einem der Stecker der Steckerbaugruppe (28) verbunden.

Wie in den Figuren 2 und 7 gezeigt, sind die innerhalb der Schlauchhülle (6) bzw. (29) aus Kunststoff angeordneten Lumina (6.1 bis 6.3) bzw. (29.1 bis 29.5) für die Zuleitungen (7, 8 und 9) der einzelnen Elektroden - bezogen auf Längsachse der Schlauchhülle - außermittig jeweils derart angeordnet, daß sowohl der kleinste Abstand zwischen der Mantelfläche der Schlauchhülle (6) bzw. (29) und der Mantelfläche der einzelnen Lumina (6.1 bis 6.3) bzw. (29.1 bis 29.5) als auch der kleinste Abstand der Mantelflächen jeweils benachbart angeordneter Lumina den gleichen Wert aufweisen.

Diese Form der Symmetrie sichert eine im wesentlichen gleichmäßige Verteilung der mechanischen Belastung der Schlauchhülle und der Zuleitungen, wenn die Elektrodenleitung bei der Implantation einer Biegebeanspruchung unterworfen wird.

Es ist ersichtlich, daß bei einem Verfahren zur Herstellung einer Defibrillationselektrodenanordnung, wie sie beispielsweise in den Figuren 1 und 6 dargestellt ist, die Ummantelung am distalen Ende der Elektrode um einen Länge gekürzt wird, welche der Länge Leiters im Bereich der Wendel bzw. der Länge des DFT-Seils als Leiter im Bereich der Wendel vermindert um die axiale Länge der Wendel entspricht, wobei das entsprechende DFT-Seil als Leiter der Zuleitung für seine Länge im Bereich der Wendel freigelegt und dann um ein die Ummantelung in axialer Richtung verlängernden zylindrischen Körper oder um den Restlängenbereich der Ummantelung, aus dem das DFT-Seil als Leiter entfernt wurde, wendelförmig herumgewickelt wird. Dabei können dann zur Herstellung einer mehrpoligen Elektrode weitere DFT-Seile als elektrische Leiter durch den Mantel der Zuleitung hindurch bzw. einer entsprechenden mechanischen Verlängerung vor dem mit der Wendel zu versehenden Ende durch die Wandung hindurchgeführt und mit aktiven Elektrodenbereichen zur Kontaktierung des Herzens verbunden werden.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Implantierbare Defibrillationselektrodenanordnung (1, 20) mit einer am distalen Ende der mit Kunststoff (6, 29) ummantelten Elektrodenleitung (2, 21) vorgesehenen Defibrillationselektrode (3, 23), wobei die Defibrillationselektrode als Schraubenwendel (3, 23) auf einem zylindrischen Träger als ein die Schraubenwendel tragender Kern ausgebildet ist, welcher an die Ummantelung der Elektrodenzuleitung anschließt oder diese fortsetzt, **dadurch gekennzeichnet, dass** die elektrische Zuleitung (8, 33) aus einem DFT-Seil besteht, welches einstückig in den aktiven Elektrodenbereich am distalen Ende der Zuleitung übergeht, wobei die Adern (13, 13') des DFT-Seils (8, 8') einen Kern (13.1, 13.1') aus einem galvanisch gut leitenden Material, bevorzugt aus Silber oder einer Silberlegierung, und einen Mantel (13.2, 13.2) aus einer Platin-Iridium-Legierung mit hoher Zugfestigkeit, aufweisen.

2. Defibrillationselektrodenanordnung nach Anspruch 1, **dadurch gekennzeichnet, dass** die Zuleitung (8, 33) durch die Schlauchhüllenwandung nach außen geführt ist und die Schlauchhülle (6, 29) den die Schraubenwendel tragenden Kern bildet.

3. Defibrillationselektrodenanordnung nach einem der Ansprüche 1 oder 2, **dadurch gekennzeichnet, dass** im Mantel der Schlauchhülle (6, 29) eine wendelförmige Nutung (6.4) zur mindestens teilweisen Aufnahme der einzelnen Windungen (3.1, 23.1) der Schraubenwendel (3, 23) vorgesehen ist.

4. Defibrillationselektrodenanordnung nach einem der vorangehenden Ansprüche, **dadurch gekennzeichnet, dass** die Wendel einlagig ausgeführt ist.

5. Defibrillationselektrodenanordnung nach Anspruch 4, 5 **dadurch gekennzeichnet, dass** die Windungen (23.1) der Schraubenwendel (23) an dem im Wendelhohlraum (23.3) geführten Abschnitt (33.1) der Zuleitung (33) jeweils eine elektrische Kontaktstelle (23.4) aufweisen.

6. Defibrillationselektrodenanordnung nach einem der vorangehenden Ansprüche, **dadurch gekennzeichnet, dass** das DFT-Seil (7, 8, 30, 31, 32, 33) sieben, im wesentlichen gleichartige, Adern (13) mit kreisförmigem Querschnittsprofil aufweist, wobei eine der Adern einen von den übrigen Adern umgriffenen Seilkern bildet.

7. Defibriltationselektrodenanordnung nach Anspruch 6, **dadurch gekennzeichnet, dass** das DFT-Seil (8') bzw. die einzelnen Adern (13') im Bereich der Schraubenwendel ein von einem Kreisquerschnitt abweichendes Querschnittsprofil aufweisen.

8. Defibrillationselektrodenanordnung nach Anspruch 7, **dadurch gekennzeichnet, dass** das Querschnittsprofil im wesentlichen elliptisch ausgebildet ist.

9. Defibrillationselektrodenanordnung nach einem der vorangehenden Ansprüche, **gekennzeichnet durch** weitere Abtast- und/oder Stimulationselektroden (4, 5, 22, 24, 25), für deren Zuleitung (7, 9, 30, 31, 32, 34) in der Schlauchhülle jeweils ein sich in axialer Richtung erstreckendes, im wesentlichen zylindrisch ausgebildetes, Lumen (6.1, 6.3, 29.1, 29.2, 29.3, 29.4) vorgesehen ist.

10. Defibrillationselektrodenanordnung nach Anspruch 9, **dadurch gekennzeichnet, dass** die als DFT-Seil ausgebildeten Zuleitungen (7, 8, 30, 31, 32, 33) in einer im wesentlichen gestreckten Form in dem jeweiligen Lumen (6.1, 6.2, 29.1, 29.2, 29.3, 29.4) angeordnet sind.

11. Defibrillationselektrodenanordnung nach einem der vorangehenden Ansprüche, **dadurch gekennzeichnet, dass** in einem Lumen (6.3, 29.5) der Schlauchhülle (6, 29) eine Stahl-Wendel (9, 34), bevorzugt aus MP35N-Stahl, als Zuleitung für eine der Elektroden (4, 22) vorgesehen ist, wobei der Wendelfreiraum (10, 35) zeitweilig einen zum Platzieren der Elektrodenleitung (2, 20) erforderlichen Führungsdraht (11, 36) aufnimmt.

12. Defibrillationselektrodenanordnung nach einem der Ansprüche 9 bis 11, **dadurch gekennzeichnet, dass** die die Zuleitungen (7 bis 9 bzw. 30 bis 34) führenden Lumina (6.1 bis 6.3 bzw. 29.1 bis 29.5) innerhalb der Schlauchhülle (6, 29) außermittig derart angeordnet sind, dass sowohl der kleinste Abstand zwischen den Mantelflächen der Schlauchhülle (6, 29) und der einzelnen Lumina (6.1 bis 6.3 bzw. 29.1 bis 29.5) als auch der kleinste Abstand der Mantelflächen jeweils benachbart angeordneter Lumina den gleichen Wert aufweisen.

13. Verfahren zur Herstellung einer Defibrillationselektrodenanordnung nach einem der vorangehenden Ansprüche, **dadurch gekennzeichnet, dass** die Ummantelung am distalen Ende der Elektrode um eine Länge gekürzt wird, welche der Länge des Leiters im Bereich der Wendel bzw. der Länge des DFT-Seils als Leiter im Bereich der Wendel vermindert um die axiale Länge der Wendel entspricht, wobei das entsprechende DFT-Seil als Leiter der Zuleitung für seine Länge im Bereich der Wendel freigelegt und dann um einen die Ummantelung in axialer Richtung verlängernden zylindrischen Körper oder um den Restlängenbereich der Ummantelung, aus dem das DFT-Seil als Leiter entfernt wurde, wendelförmig herumgewickelt wird.

14. Verfahren zur Herstellung einer Defibrillationselektrodenanordnung nach Anspruch 14, **dadurch gekennzeichnet, dass** zur Herstellung einer mehrpoligen Elektrode weitere DFT-Seile als elektrische Leiter durch den Mantel der Zuleitung hindurch bzw. einer entsprechenden mechanischen Verlängerung vor dem mit der Wendel zu versehenden Ende durch die Wandung hindurchgeführt und mit aktiven Elektrodenbereichen zur Kontaktierung des Herzens verbunden werden.

## Claims

1. An implantable defibrillation electrode arrangement (1, 20) comprising a defibrillation electrode (3, 23) provided at the distal end of the electrode line (2, 21) which is encased with plastic material (6, 29), wherein the defibrillation electrode is in the form of a helical coil (3, 23) on a cylindrical carrier in the form of a core which carries the helical coil and which adjoins or extends the casing of the electrode feed line, **characterised in that** the electrical feed line (8, 33) comprises a DFT cable which forms a transition integrally into the active electrode region at the distal end of the feed line, wherein the wires (13, 13') of the DFT cable (8, 8') have a core (13.1, 13.1') of a material which is a good galvanic conductor, preferably of silver or a silver alloy, and a sheath (13.2, 13.2) of a platinum-iridium alloy with a high level of tensile strength.

2. A defibrillation electrode arrangement according to claim 1 **characterised in that** the feed line (8, 33) is passed outwardly through the tube sheathing wall and the tube sheathing (6, 29) forms the core carrying the helical coil.

3. A defibrillation electrode arrangement according to one of claims 1 and 2 **characterised in that** provided in the peripheral surface of the tube sheathing (6, 29) is a helical grooving (6.4) for at least partially receiving the individual turns (3.1, 23.1) of the helical coil (3, 23).

4. A defibrillation electrode arrangement according to one of the preceding claims **characterised in that** the coil is of a single-layer configuration.

5. A defibrillation electrode arrangement according to claim 4 and claim 5 **characterised in that** the turns (23.1) of the helical coil (23) have a respective electrical contact location (23.4) at the portion (33.1) of the feed line (33), which is guided in the coil cavity (23.3).

6. A defibrillation electrode arrangement according to one of the preceding claims **characterised in that** the DFT cable (7, 8, 30, 31, 32, 33) has seven substantially similar wires (13) of circular cross-sectional profile, wherein one of the wires forms a cable core which is embraced by the other wires.

7. A defibrillation electrode arrangement according to claim 6 **characterised in that** the DFT cable (8') or the individual wires (13') in the region of the helical coil are of a cross-sectional profile which differs from a circular cross-section.

8. A defibrillation electrode arrangement according to claim 7 **characterised in that** the cross-sectional profile is substantially elliptical.

9. A defibrillation electrode arrangement according to one of the preceding claims **characterised by** further sensing and/or stimulation electrodes (4, 5, 22, 24, 25), for the feed line (7, 9, 30, 31, 32, 34) of which there is provided in the tube sheathing a respective lumen (6.1, 6.3, 29.1, 29.2, 29.3, 29.4) which extends in the axial direction and which is substantially cylindrical.

10. A defibrillation electrode arrangement according to claim 9 **characterised in that** the feed lines (7, 8, 30, 31, 32, 33) in the form of the DFT cable are arranged in a substantially straight form in the respective lumen (6.1, 6.2, 29.1, 20.2, 29.3, 29.4).

11. A defibrillation electrode arrangement according to one of the preceding claims **characterised in that** provided in a lumen (6.3, 29.5) of the tube sheathing (6, 29) is a steel coil (9, 34), preferably of MP35N steel, as a feed line for one of the electrodes (4, 22), wherein the coil free space (10, 35) temporarily accommodates a guide wire (11, 36) required for placement of the electrode line (2, 20).

12. A defibrillation electrode arrangement according to one of claims 9 to 11 **characterised in that** the lumens (6.1 to 6.3 and 29.1 to 29.5) which guide the feed lines (7 to 9 and 30 to 34) are arranged eccentrically within the tube sheathing (6, 29) in such a way that both the smallest spacing between the peripheral surfaces of the tube sheathing (6, 29) and the individual lumens (6.1 to 6.3 and 29.1 to 29.5) and also the smallest spacing of the peripheral surfaces of respectively adjacently arranged lumens are of the same value.

13. A process for the production of a defibrillation electrode arrangement according to one of the preceding claims **characterised in that** the casing at the distal end of the electrode is shortened by a length which corresponds to the length of the conductor in the region of the coil or the length of the DFT cable as a conductor in the region of the coil reduced by the axial length of the coil, wherein the corresponding DFT cable is exposed as the conductor of the feed line for its length in the region of the coil and then is wound in a coil configuration around a cylindrical body prolonging the casing in the axial direction or around the remaining lengthwise region of the casing from which the DFT cable was removed as the conductor.

14. A process for the production of a defibrillation electrode arrangement according to claim 13 **characterised in that** to produce a multipole electrode further DFT cables as electrical conductors are passed through the peripheral surface of the feed line or a corresponding mechanical prolongation before the end to be provided with the coil through the wall thereof and are connected to active electrode regions for contacting the heart.

## Revendications

1. Dispositif implantable à électrodes de défibrillation (1,20) comportant une électrode de défibrillation (3,23) prévue sur l'extrémité distale de la ligne des électrodes (2,21) enveloppée d'une matière plastique (6, 29), dans lequel l'électrode de défibrillation est agencée sous la forme d'un filament hélicoïdal (3,23) disposé sur un support cylindrique comme une âme portant le filament hélicoïdal et qui se raccorde à la gaine de la ligne d'alimentation des électrodes ou prolonge cette dernière, **caractérisé en ce que** la ligne d'alimentation électrique (8,33) est constituée par un câble DFT, qui se prolonge d'un seul tenant par la zone d'électrode active sur l'extrémité distale de la ligne d'alimentation, les conducteurs (13, 13') du câble DFT (8,8') portant une âme (13.1,13.1') formé par un matériau bon conducteur du point de vue galvanique, de préférence en argent ou en un alliage d'argent, et une enveloppe (13.2,13.2) formée d'un alliage platine-iridium présentant une résistance élevée à la traction.

2. Dispositif à électrodes de défibrillation selon la revendication 1, **caractérisé en ce que** la ligne d'alimentation (8,33) est guidée extérieurement par la paroi de la gaine en forme de tuyau (6, 29) forme l'âme portant le filament hélicoïdal.

3. Dispositif à électrodes de défibrillation selon l'une des revendications 1 ou 2, **caractérisé en ce que** dans la gaine de la gaine en forme de tuyau (6,29) est prévue un rainurage de forme hélicoïdale (6.4) servant à loger au moins partiellement les différentes spires (3.1,23.1) du filament hélicoïdal (3,23).

4. Dispositif à électrodes de défibrillation selon l'une des revendications précédentes, **caractérisé en ce que** le filament hélicoïdal est agencé sous la forme d'une seule couche.

5. Dispositif à électrodes de défibrillation selon l'une des revendications 4, 5, **caractérisé en ce que** les spires (23.1) du filament hélicoïdal (23) comportent respectivement un point de contact électrique (23.4), dans la section (33.1), guidée dans la cavité à filament (23.3) de la ligne d'alimentation (33).

6. Dispositif à électrodes de défibrillation selon les revendications précédentes, **caractérisé en ce que** le câble DFT (7,8,30,31,32,33) comporte sept conducteurs (13) sensiblement identiques, possédant un profil circulaire en coupe transversale, l'un des conducteurs formant une âme du câble entouré par les autres conducteurs.

7. Dispositif à électrodes de défibrillation selon la revendication 6, **caractérisé en ce que** le câble DFT (8') et les différents conducteurs (13') possèdent dans la zone du filament hélicoïdal, un profil en coupe transversale qui diffère d'une section transversale circulaire.

8. Dispositif à électrodes de défibrillation selon la revendication 7, **caractérisé en ce que** le profil en coupe transversale est agencé avec une forme essentiellement elliptique.

9. Dispositif à électrodes de défibrillation selon l'une des revendications précédentes, **caractérisé par** les autres électrodes d'exploration par balayage et/ou de stimulation (4,5,22,24,25), pour la ligne d'alimentation (7, 9,30,31,32,33) desquelles il est prévu, dans la gaine en forme de tuyau, respectivement une lumière (6.1,6.3,29.1,29.2, 29.3,29.4) qui s'étend dans la direction axiale et est réalisée avec une forme essentiellement cylindrique.

10. Dispositif à électrodes de défibrillation selon la revendication 9, **caractérisé en ce que** les lignes d'alimentation (7,8,30,31,32,33) réalisées sous la forme d'un câble DFT sont disposées sous une forme sensiblement étirée dans la lumière respective (6.1,6.2,29.1,29.2,29.3, 29.4).

11. Dispositif à électrodes de défibrillation selon l'une des revendications précédentes, **caractérisé en ce que** dans une lumière (6.3,29.5) de la gaine en forme de tuyau (6,29) est prévu un filament en acier (9,34), de préférence en acier MP35N, en tant que ligne d'alimentation pour l'une des électrodes (4,22), l'espace (10,35) pour les filaments logeant par instants un fil de guidage (11,36) nécessaire pour la mise en place de la ligne d'électrodes (2,20).

12. Dispositif à électrodes de défibrillation selon l'une des revendications 9 à 11, **caractérisé en ce que** les lumières (6.1 à 6.3 ou 29.1 à 29.5), qui guident les lignes d'alimentation (7 à 9 ou 30 à 34) sont disposées d'une manière décentrée à l'intérieur de la gaine en forme de tuyau (6,29) de telle sorte qu'aussi bien la plus petite distance entre la surface d'enveloppe de la gaine en forme de tuyau (6,29) et les différentes lumirèes (6.1 à 6.3 ou 29.1 à 29.5) que la plus petite distance des surfaces enveloppes de lumière respectivement voisines possèdent la même valeur.

13. Procédé pour fabriquer un dispositif à électrodes de défibrillation selon l'une des revendications précédentes, **caractérisé en ce que** l'enveloppement réalisé sur l'extrémité distale de l'électrode est raccourci d'une longueur qui correspond à la longueur du conducteur dans la zone du filament hélicoïdal ou à la longueur du câble DFT en tant que conducteur dans la zone du filament hélicoïdal, diminuée de la longueur axiale du filament hélicoïdal, le câble DFT correspondant étant disposé librement en tant que conducteur de la ligne d'alimentation sur sa longueur dans la zone du filament hélicoïdal et étant enroulé sous la forme d'un filament hélicoïdal autour d'un corps cylindrique, qui prolonge l'enveloppement dans la direction axiale, ou autour de la zone de longueur résiduelle de l'enveloppement, dont le câble DFT a été retiré en tant que conducteur.

14. Procédé pour fabriquer un dispositif à électrodes de défibrillation selon la revendication 13, **caractérisé en ce que** pour la fabrication d'une électrode multipolaire, on fait passer d'autres conducteurs DFT en tant que conducteurs électriques à travers l'enveloppe de la ligne d'alimentation ou un prolongement mécanique correspondant en amont de l'extrémité devant être pourvue du filament, à travers la paroi et on le relie à des parties actives d'électrode pour l'établissement du contact avec le coeur.
